Europäisches Patentamt

European Patent Office

Office européen des brevets

(11) Veröffentlichungsnummer: **0 485 652 A1**

## (12) EUROPÄISCHE PATENTANMELDUNG

(21) Anmeldenummer: **90121753.9**

(22) Anmeldetag: **14.11.90**

(51) Int. Cl.⁵: **A61K 39/00**

(43) Veröffentlichungstag der Anmeldung:
**20.05.92 Patentblatt 92/21**

(84) Benannte Vertragsstaaten:
**AT BE CH DE ES FR GB IT LI LU NL SE**

(71) Anmelder: **Theurer, Karl E., Prof.Dr.med.**
**Brunnwiesenstrasse 21**
**W-7302 Ostfildern 1(DE)**

(72) Erfinder: **Theurer, Karl E., Prof.Dr.med.**
**Brunnwiesenstrasse 21**
**W-7302 Ostfildern 1(DE)**

(54) **Verfahren zur Herstellung von Impfvaccinen gegen Krankheitserreger.**

(57) Die Substrate werden dabei ggfs. im Vakuum Dämpfen von Persäuren oder von Mischungen von Persäuren und den entsprechenden konzentrierten Säuren ausgesetzt und die Produkte, wenn erforderlich, fraktioniert und zusammen mit Adjuvantien zur Gewinnung von Antikörpern, katalytischen Antikörpern, Abwehr-Proteinasen bzw. sensibilisierten T-Lymphozyten verwendet.

EP 0 485 652 A1

Rank Xerox (UK) Business Services
(−/2.19/2.0)

Die Erfindung betriff ein Verfahren zur Gewinnung von Impfvaccinen zur Schutzimpfung und Therapie gegen Infektionen mit Mikroorganismen, Parasiten konventionellen und unkonventionellen Viren (Viroiden, Prionen) sowie Toxine und Tumore. Sie beruht auf dem Verfahren zur schonenden Sterilisation von biologischen Wirkstoffen, insbesondere von Organgeweben für therapeutische Zwecke gegenüber Mikroorganismen und Viren (DE 2944278; EU. Patent Nr. 0153995), bei dem man die Substrate im Vakuum Dämpfen von Persäuren oder von Mischungen von Persäuren und den entsprechenden konzentrierten Säuren aussetzt, wobei diese ein Mischungsverhältnis von 1 Teil Persäure zu 1 bis 4 Teilen des entsprechenden Säureanhydrids aufweisen. Der Vorteil dieser Mischung ist, daß die partiellen Persäuren weniger aggressiv und besser manipulierbar, d.h. weniger explosiv sind.

Es konnte festgestellt werden, daß durch dieses Verfahren Mikroorganismen und die verschiedenen Arten der genannten Viren auch bei Anreicherung abgetötet bzw. inaktiviert werden unter Beibehaltung der immunogenen Eigenschaften, sodaß diese als Impfvaccine verwendet werden können. Die kontinuierliche steuerbare Einwirkung der Persäuredämpfe auf Proteine, Nucleinsäuren, Lipide und Kohlenhydrate führt zu deren Hydrolyse, d.h. zur Spaltung von komplizierten organischen Verbindungen in einfachere und zu deren Oxydation. Das Ausmaß dieser Veränderungen hängt von der Dampfkonzentration, vom Mischungsverhältnis von Wasserstoffsuperoxyd und Säureanhydrid, von der Einwirkungszeit und der Temperatur ab, sodaß bei geringgradiger Einwirkung der Persäuredämpfe möglicherweise auch abgeschwächte Lebendvaccine gewonnen werden können.

Die Oxydation verhindert die Rekombination von Nucleinsäurefragmenten und die Reaggregation von Peptiden. Durch das Verfahren werden aus dem Substrat Epitope freigelegt und möglicherweise zusätzliche antigene Determinanten erschlossen. Die hydrolytische Spaltung führt also trotz verkleinerter Moleküle zu einer Erweiterung des antigenen Spektrums. Zur Absicherung gegen Infektiosität des Persäure-Lysats kann dieses einer Ultrafiltration auf Molekulargewichte unterhalb der nativen Infektionserreger unterzogen werden.

Es ist anzunehmen, daß die Persäurebedampfung der Substrate bei deren prophylaktischer und therapeutischer Anwendung zusätzlich zur Antikörperbildung auch zu einer Bildung von Abwehrproteinasen führt. Diese hat Rudolf ABDERHALDEN für Organpräparate nachgewiesen, die mit Säuredämpfen behandelt waren. Abwehrproteinasen können im Gegensatz zu induzierten Antikörpern zum direkten Abbau von Proteinen der Infektionserreger führen und so den prophylaktischen und therapeutischen Effekt von Impfvaccinen verbessern. Abwehrproteinasen sind möglicherweise identisch mit katalytischen Antikörpern mit der Fähigkeit von Enzymen (vgl.: Richard A. LERNER und Alfons TRAMONTANO: Spektrum der Wissenschaft, Mai 1988, S. 78 u.f.). Die Persäurebedampfung des Substrats erzeugt Übergangszustände bei der Fragmentierung und begünstigt die Bildung solcher katalytischer Antikörper, die zusätzlich stereochemisch unterschiedliche Formen eines Moleküls mit verschiedener Chiralität unterscheiden können und somit die Abwehrfähigkeit des Organismus erweitern und verstärken. Dieser Effekt ist bei den bisherigen Herstellungsverfahren von Vaccinen unwahrscheinlich und deshalb überraschend.

Die biologisch-medizinische Wirkung der durch Persäurebedampfung gewonnenen Vaccine kann nach bekannten Methoden durch Adjuvantien verstärkt werden. Auch lassen sich Mischvaccine herstellen oder aber die Vaccine in getrennte Fraktionen unterteilen und zur Gewinnung von bestimmten Antikörpern, katalytischen Antikörpern, Abwehrproteinasen oder sensibilisierten T-Lymphozyten, einzeln oder in Mischung verwenden.

Das Verfahren zur Gewinnung solcher Impfvaccine ist im Patentanspruch beschrieben. Man geht dabei von pulverisierten Substraten der Krankheitserreger mit einem Restfeuchtigkeitsgehalt von 3 bis 30% aus, die eine Hydrolyse und Oxydation mit Persäuredämpfen erlaubt. Auch kann man von tiefgekühlten und pulverisierten oder suspendierten oder gelösten Substraten ausgehen und nach einleitender Lyophylisation Persäuredampf in den Rezipienten in vorbestimmter Menge einleiten und durch Absenken des Vakuums zunächst auf dem Substrat niederschlagen und nach der gewünschten Einwirkung das überschüssige Reagenz durch Erhöhung des Vakuums wieder entfernen. Eine chemische Neutralisierung des Substrats erübrigt sich dadurch. Der Kondensationsvorgang kann, wenn erforderlich, mehrmals wiederholt werden. Der Grad der Hydrolyse läßt sich durch Vergleich des Molekulargewichts bestimmen, wodurch eine Standardisierung möglich wird. Der Verlust der pathogenen Eigenschaften des Substrats, die Sterilität und biologische Wirkung muß separat geprüft werden. Weitere Angaben darüber sind aus den genannten Patentschriften des Verfahrens zur schonenden Sterilisation von biologischen Wirkstoffen zu entnehmen.

Die Technik der Einwirkung der Persäuredämpfe auf das Substrat kann variiert werden von der planen Ausbreitung derselben im Dünnschichtverfahren zu Verfahren vergrößerter Oberfläche, z.B. im Rotationsverdampfer, durch Schüttel- und Rüttelverfahren, ggfs. über Transportbänder mit freiem Fall der Pulver sowie durch Sprühverfahren bei unterschiedlichen Temperaturen, die nicht zur Denaturierung führen. Dadurch können größere Mengen des Substrates bedampft werden, wie dies bei der Sterilisation von Tierfutter geschieht. Das Verfahren der Bedampfung mit Persäuren muß den jeweiligen Gegebenheiten angepaßt werden. Eine zuweitgehende Lyse vernichtet die spezifische Schutzwirkung.

Die Herstellung von Impfvaccinen durch Bedampfung von Krankheitserregern mit Persäuren schließt Parasiten und Toxine, sowie Tumore mit ein. In der Erschließung von Epitopen, die als antigene Determinanten vorher nicht zugänglich sind, besteht der grundlegende Unterschied zum enzymatischen Abbau auch von Molekülen. Mit an Sicherheit grenzender Wahrscheinlichkeit werden auch in variablen Antigenen stabile Regionen des Antigens frei und für eine Schutzwirkung zugänglich.

Beispiel 1

Es sollen Impfvaccine durch Bedampfung mit Persäuren aus Streptokokken gewonnen werden. Die Mikroorganismen werden in Kultur gezüchtet und nach bekannten Methoden isoliert und in wässerigem Suspensionsmittel angereichert, sodann tiefgefroren, pulverisiert und in einen Rotationsverdampfer gebracht. Dort kann durch Lyophilisation oder über konzentrierter Schwefelsäure im Vakuum zunächst die Trocknung bis zu einem Restfeuchtigkeitsgehalt von etwa 5 bis 10% erfolgen. Das Trocknungsmittel kann sich in einem kommunizierenden Gefäß befinden. Der Eksikkator des Rotationsverdampfers steht über einen Dreiwegehann mit der Hochvakuumpumpe und einem Druckgefäß, in dem sich 10 ml einer Mischung von 1 Teil Peressigsäure und 2 Teilen Essigsäure befinden, in Verbindung. Nach dem Trocknungsvorgang wird die Vakuumpumpe durch das Ventil abgeschaltet und der Rezipient mit Persäuremischung zum Eksikkator geöffnet, sodaß die Säure verdampft und in den Eksikkator gelangt. Es hängt vom Volumen des Exsikkators ab, wie viel Säure verdampft. Ggfs. muß wiederholt das Vakuum erneuert werden, damit die gesamte Säure verdampfen kann. Die Einwirkungszeit richtet sich nach dem Grad der Lyse des Substrates bzw. dessen Sterilität und kann je nach Menge des Substrates Bruchteile von Stunden bis Tage benötigen. Nach der Säureeinwirkung kann der Rezipient für Säure durch einen solchen mit Merkaptoäthanol ersetzt werden und dieser in gleicher Weise, wie vorher die Persäure, auf das Substrat zur Einwirkung gebracht werden, bevor man den Trocknungsvorgang durch erneutes Absaugen beendet. Eine chemische Neutralisierung des Substrats erfolgt weitgehend bereits durch das Absaugen im Vakuum. Das gewonnene Trockenpulver erweist sich bei bakteriologischer und virologischer Überprüfung als steril. Zur Verbesserung seiner immunologischen Wirksamkeit kann es vor der endgültigen Trocknung mit einem Adjuvans imprägniert werden, z.B. mit einer kolloidalen Komplexverbindung aus Aluminiumhydroxyd und Kieselsäure sowie einem nicht denaturierendem Konservierungsmittel, z.B. Antibiotikum oder Chemotherapeutikum. Die Abpackung kann in immunogenen Dosen, z.B. in Konzentrationen von $10^{-4}$ g Trockenpulver mit beigegebenem Suspensionsbzw. Lösungsmittel oder als Lösung bzw. Suspension erfolgen.

Auf analoge Weise werden auch gegen andersartige Mikroorganismen und Parasiten Impfvaccine gewonnen. Bei Parasiten können Präparate aus den verschiedenen zyklischen Lebensformen, z.B. Sporozoiten, Schizonten, Merozoiten, Gametozyten und Oozyten einzeln und als Mischvaccine hergestellt werden.

Beispiel 2

Zur Herstellung von Impfvaccinen gegen Toxine werden Lösungen von diesen lyophilisiert und pulverisiert, sodann den Dämpfen von Persäuren ausgesetzt und entsprechend dem Beispiel 1 weiterverarbeitet.

Beispiel 3

Die Herstellung von Impfvaccinen gegen konventionelle und unkonventionelle Viren erfolgt aus isolierten und angereicherten Viren, Virionen, Viroiden oder Prionen. Auch hier wird die Bedampfung mit Persäuren bzw. Mischungen von Persäuren mit den entsprechenden konzentrierten Säuren auf das pulverisierte Substrat mit einem Restfeuchtigkeitsgehalt durchgeführt und geeignete Adjuvantien und ggfs. schonende Konservierungsmittel mitverwendet.

Der Weg zu einer zuverlässigen sicheren Vaccine, nicht nur gegen HIV und unkonventionellen Viren, ist die Erschließung von antigenen Epitopen, die sonst nicht zugänglich sind. Es ist höchst wahrscheinlich, daß persistierende, sich nicht ständig verändernde Antigenqualitäten erreicht werden. Mischvaccine gegen verschiedene Virusstämme und verwandte Arten erscheinen zweckmäßig.

Beispiel 4

Herstellung von Impfvaccinen gegen Tumore - Versuche an Mäuse-Inzuchtstämmen führte zur Entdeckung, daß Tumore spezifische Antigene aufweisen, die sich bei verschiedenen Tumorarten unterscheiden und auf der Expression von Tumorgenen beruhen. Auch Tumorviren sind daran beteiligt. Es können deshalb nur tumorspezifische Vaccine gewonnen werden oder aber Mischvaccine gegen verschiedene Tumore der gleichen Art und/oder gegenüber unterschiedlichen Arten. Das Ausgangsmaterial soll möglichst von nicht tumorösem Gewebe freipräpariert werden, bevor es tiefgefroren und evtl. später pulverisiert und den Dämpfen von Persäuren ausgesetzt wird. Auch hier empfiehlt sich die Mitverwendung von Adjuvantien und Konservierungsmittel.

**Patentansprüche**

1. Verfahren zur Herstellung von Impfvaccinen gegen Mikroorganismen, Parasiten, konventionellen und unkonventionellen Viren (Viroiden und Prionen), Toxine sowie Tumore analog einem Verfahren zur schonenden Sterilisation von biologischen Wirkstoffen, insbesondere von Organgeweben für therapeutische Zwecke gegenüber Mikroorganismen und Viren (DE 2944278; EU. Patent Nr. 0153995), bei welchem man die pulverförmigen Substrate im Vakuum Dämpfen von Persäuren aus Essigsäure, Ameisensäure, Schwefelsäure oder Diäthylamin in einem abgeschlossenen System zusammen, aber getrennt voneinander, einem solchen Vakuum aussetzt, welches die Überführung des aufschließenden Mittels in die dampfförmige Phase ermöglicht und nachdem der gewünschte Aufschluß des Substrates erreicht ist, das nicht verbrauchte Reagenz wieder entfernt, wobei man insbesondere von tiefgekühlten und pulverisierten organischen Geweben ausgeht und man das dampfförmige chemische Reagenz aus einer vorbestimmten Menge durch Absenkung des Vakuums zunächst auf dem Substrat niederschlägt, und nach der gewünschten Einwirkung das überschüssige Reagenz durch Erhöhung des Vakuums wieder entfernt, wobei die Säuredämpfe ein Mischungsverhältnis von 1 Teil Persäure : 1 bis 4 Teilen des entsprechenden Säureanhydrids aufweisen können,

   dadurch gekennzeichnet, daß pathogenes Material, das durch die bekannten Methoden der Mikrobiologie oder Virologie durch Züchtung von Mikroorganismen oder Viren bzw. aus Tumorgewebe in angereicherter oder isolierter Form gewonnen wird, der Einwirkung von Persäuren in dampfförmigem Zustand bei Temperaturen, die nicht zur Denaturierung führen, ausgesetzt und nach Prüfung auf Verlust der Infektiosität, ggfs. zusammen mit einem immunologischem Adjuvans, zu Impfstoffen verarbeitet wird.

2. Verfahren nach Anspruch 1, dadurch gekennzeichnet, daß durch milde Persäureeinwirkung abgeschwächte Lebendvaccine gewonnen werden.

3. Verfahren nach Anspruch 1, dadurch gekennzeichnet, daß die Produkte in getrennte Fraktionen unterteilt oder daß Mischvaccine hergestellt werden.

4. Verfahren nach Anspruch 1, 2 und 3, dadurch gekennzeichnet, daß die Produkte zur Gewinnung von Antikörpern, katalytischen Antikörpern, Abwehrproteinasen bzw. sensibilisierten T-Lymphozyten, einzeln oder in Mischung verwendet werden.

Europäisches Patentamt

# EUROPÄISCHER RECHERCHENBERICHT

Nummer der Anmeldung

EP 90 12 1753

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | WO-A-8 707 507 (R. SEKURA) * Das ganze Dokument * --- | 1-4 | A 61 K 39/00 |
| Y,D | EP-A-0 153 995 (K. THEURER) * Das ganze Dokument * --- | 1-4 | |
| A | DE-A-1 617 865 (K. THEURER) * Das ganze Dokument * --- | 1-4 | |
| A | EP-A-0 029 893 (K. THEURER) * Das ganze Dokument * ----- | 1-4 | |

**RECHERCHIERTE SACHGEBIETE (Int. Cl.5)**

A 61 K
C 12 N

Der vorliegende Recherchenbericht wurde für alle Patentansprüche erstellt

| Recherchenort | Abschlußdatum der Recherche | Prüfer |
|---|---|---|
| DEN HAAG | 06-06-1991 | FERNANDEZ Y BRANAS F.J. |

EPO FORM 1503 03.82 (P0403)